# EUROPÄISCHE PATENTANMELDUNG

(11) **EP 1 736 117 A2**
(43) Veröffentlichungstag der Anmeldung: **27.12.2006**
(21) Anmeldenummer: 05014245.4
(22) Anmeldetag: 30.06.2005
(51) Int. Cl.: A61F 2/16

(54) **Vorrichtung zum Laden von Intraokularlinsen in ein Injektionssystem**

(30) Priorität: 10.06.2005 DE 202005009089 U
(71) Anmelder: Acrimed GmbH, 13507 Berlin (DE)
(72) Erfinder: Klemm, Frank Dr., 16565 Lehnitz (DE)
(74) Vertreter: Schildberg, Peter

(57) **Zusammenfassung**

Vorrichtung zum Laden von Intraokularlinsen in ein Injektionssystem, mit
- einer Kartusche, die zwei Flügelelemente (14,16) aufweist, von denen mindestens eines der beiden schwenkbar angelenkt ist, die Flügelelemente bilden in einer geschlossenen Position einen Linsenkanal für die gefaltete Intraokularlinse,
wobei
ein Sperrelement (30) vorgesehen ist, das mit den Flügelelementen zusammenwirkt, um die Kartusche in einer Position mit geöffneten Flügelelementen zu halten und
das Sperrelement einen Griffabschnitt (32) und einen Halteabschnitt (34,36) besitzt.

## Beschreibung

Die vorliegende Erfindung betrifft eine Vorrichtung zum Laden von Intraokularlinsen in ein Injektionssystem. Faltbare Intraokularlinsen werden mit Hilfe von Injektionssystemen in das Auge eingebracht, wo sie sich nachfolgend entfalten können. Wichtig für das Einsetzen mit Hilfe eines Injektionssystem ist, daß die Intraokularlinse richtig gefaltet und steril in das Injektionssystem eingebracht wird. Dieser Vorgang wird als Laden des Injektionssystems mit einer Intraokularlinse bezeichnet.

Aus WO 03/045285 A1 ist ein Set zum Implantieren einer Intraokularlinse bekannt. Das Set besteht aus einer Kartusche, die mit zwei Flügelelementen versehen ist. Die Flügelelemente sind mittig über ein Filmscharnier miteinander verbunden und besitzen in einem ersten Abschnitt, der sich an das Gelenk anschließt, eine gewölbte Wand, die in einen geraden Wandabschnitt übergeht. Zur spannungsfreien Lagerung der Intraokularlinse in der Kartusche ist ein Halter vorgesehen, der beispielsweise als Stopfen auf eine Flasche gesetzt werden kann. Der Halter besitzt eine Öffnung, die so bemessen ist, daß der Linsenträger in die Öffnung eingesetzt die Linse spannungsfrei hält. Um die in die Öffnung eingesetzten Linsenträger in der Haltevorrichtung zu sichern, wird ein Sicherungselement zwischen die Flügel des Linsenhalters eingeführt. Der Linsenhalter selbst besitzt an der Außenseite der Flügelelemente wiederum abstehende Vorsprünge, die eine Vertiefung in der Haltevorrichtung hintergreifen. Im gesicherten Zustand ist also der Linsenhalter in einer weitgehend geschlossenen Position formschlüssig mit der Halteeinrichtung verbunden. Zur Benutzung der Linse ist es erforderlich, durch Zusammendrücken der Flügelelemente das Sicherungselement zu entfernen und die Kartusche aus der Halteeinrichtung zu entfernen.

Aus DE 20 2004 009 556.4 ist ein System bestehend aus Kartusche und Haltevorrichtung bekannt. Die Kartusche verfügt über ein hohles Führungsrohr, das den Linsenkanal für die gefaltete Intraokularlinse bildet. Die Haltevorrichtung besitzt eine Aufnahme für das Führungsrohr und seitlich unter einem Winkel angeordnete Schlitze, die im eingesetzten Zustand die Flügel der Kartusche halten. Mit dieser Haltevorrichtung ist es möglich, die Kartusche mit ganz oder teilweise gespreizten Flügelelementen zu halten. Nachteilig an der bekannten Haltevorrichtung ist jedoch, daß die Kartusche sich nur ungenau aus der Haltevorrichtung entnehmen läßt.

Der Erfindung liegt die Aufgabe zugrunde, ein System zum Laden von Intraokularlinsen für ein Injektionssystem bereitzustellen, das mit einfachen Mitteln eine zuverlässige Faltung der Linse erlaubt.

Erfindungsgemäß wird die Aufgabe durch eine Vorrichtung mit den Merkmalen aus Anspruch 1 gelöst.

Die erfindungsgemäße Vorrichtung besteht aus einer Kartusche und einem Sperrelement. Die Kartusche besitzt zwei Flügelelemente, von denen mindestens eines der beiden schwenkbar gelagert ist. Die Flügelelemente bilden in einer geschlossenen Position einen Linsenkanal für eine gefaltete Intraokularlinse. Erfindungsgemäß ist ein Sperrelement vorgesehen, das mit den Flügelelementen zusammenwirkt, um die Kartusche in einer Position mit aufgeschwenkten Flügelelementen zu halten. Das Sperrelement besitzt einen Griffabschnitt und einen Halteabschnitt, die bevorzugt beide flach ausgebildet sind. Der Halteabschnitt wirkt mit den Flügelelementen zusammen, um diese in einer geöffneten Winkelposition zu halten. Die geöffnete Winkelposition der Kartusche ist dabei bevorzugt so gewählt, daß eine Intraokularlinse spannungsfrei von den Flügelelementen der Kartusche gehalten wird. Das Sperrelement besitzt ferner einen Griffabschnitt, der es ermöglicht, die Kartusche mit der Linse zu greifen. Das erfindungsgemäße System besitzt den Vorzug, daß die Handhabung der geöffneten Kartusche mit der Linse durch das Sperrelement deutlich erleichtert wird. So kann beispielsweise der Operateur die Kartusche der Verpackung entnehmen, die Linse mit Gleitmittel versehen und nach Entfernen des Sperrelements die Linse durch Schließen der Kartusche zusammenfalten.

Bevorzugt besitzt der Halteabschnitt an einer Seite des Griffabschnitts mindestens zwei Paare von Fingern, von denen jeweils ein Paar ein Flügelelement hält. Die Paare von Fingern besitzen jeweils einen geraden, länglichen Schlitz, mit dem das Sperrelement auf die Flügelelemente der Kartusche aufgesteckt und/oder von diesen abgezogen werden kann. Das Sperrelement kann, beispielsweise nachdem die Intraokularlinse mit Flüssigkeit bedeckt oder die Lage der Intraokularlinse vor dem Falten kontrolliert wurde, zusammengefaltet werden, indem das Sperrelement von den Flügeln der Kartusche abgezogen wird. Der zwischen den Fingern eines Paares gebildete Schlitz besitzt an mindestens einem Finger eine Anlagefläche, die im aufgesteckten Zustand flächig an dem Flügelelement anliegt. Mindestens einer der Finger ist derart ausgebildet, dass eine Bewegung der in die Kartusche eingelegten Linse in Richtung Sperrelement verhindert wird.

In einer besonders bevorzugten Ausgestaltung der Erfindung ist zusätzlich zu der Kartusche und dem Sperrelement noch ein wannenförmiger Behälter vorgesehen. Der Behälter dient zum Transport der Kartusche mit einer eingesetzten Intraokularlinse. Einen wannenförmigen Behälter zu verwenden, bietet den Vorteil, daß die Kartusche mit der Intraokularlinse in einer Flüssigkeit gelagert werden kann.

Der Behälter besitzt vorzugsweise zwei Ablageflächen für die Flügelelemente in der durch das Sperrelement vorgegebenen Position. Die Ablageflächen besitzen zweckmäßigerweise Halteabschnitte für die Flügelelemente, so daß die Kartusche unbeweglich in dem Behälter gehalten ist. Die Ablagefläche ist zweckmäßigerweise in einer wannenförmigen Vertiefung angeordnet derart, daß für eine auf den Ablageflächen angeordnete Kartusche sowohl eine Kartuschenspitze als auch der Griffabschnitt seitlich über die Ablageflächen vorstehen. Bevorzugt ist der Behälter einstückig geformt und wird zweckmäßigerweise durch eine Abdeckung verschlossen. Bevorzugt ist als Abdeckung eine vom Behälterkörper abziehbare Folie vorgesehen.

Als besonders vorteilhaft hat es sich herausgestellt, die Flügelelemente an ihren einander zugewandten Seiten mit einem Verschluß zu versehen. Die Lage des Verschlusses auf den Seitenflächen gibt der Bedienperson eine bessere haptische Kontrolle beim Schließen der Kartusche. Bevorzugt besitzt der Verschluß mindestens einen Vorsprung und mindestens eine Vertiefung, die in der verschlossenen Position der Kartusche ineinander angeordnet sind. Vorsprung und Vertiefung bilden eine form- und/oder kraftschlüssige Verbindung zwischen den Flügeln.

Zweckmäßigerweise erstreckt sich der Vorsprung entlang dem Flügelelement parallel zu dessen Schwenkachse. Das Flügelelement besitzt einen kreisförmig gewölbten Abschnitt, der in einen ebenen Abschnitt übergeht. In der geschlossenen Position bilden die kreisförmigen Abschnitte einen Linsenkanal, durch den die gefaltete Linse mit dem Injektionssystem in das Auge gefördert wird.

Bevorzugt ist die Kartusche mit einer Spitze versehen, die den von den Flügelelementen gebildeten Linsenkanal verlängert.

Ein bevorzugtes Ausführungsbeispiel des erfindungsgemäßen Systems wird anhand der nachfolgenden Figuren näher erläutert.

Es zeigt:
- Fig. 1: eine Kartusche mit eingesetztem Sperrelement und Linse in einer perspektivischen Ansicht von vorne,
- Fig. 2: die Kartusche mit Sperrelement in einer perspektivischen Ansicht von hinten,
- Fign. 3 u. 4: einen Behälter für die Kartusche in einer perspektivischen Ansicht von schräg oben,
- Fig. 5: die geöffnete Kartusche mit eingesetzer Linse ohne Sperrelement,
- Fig. 6: den Behälter für die Kartusche in einer Schnittansicht,
- Fig. 7: das Sperrelement 30 in einer Draufsicht,
- Fig. 8: das Sperrelement aus Fig. 7 in einem Schnitt entlang der Linie VIII - VIII,
- Fig. 9: eine Seitenansicht des Sperrelements und
- Fig. 10: eine perspektivische Ansicht des Sperrelements.

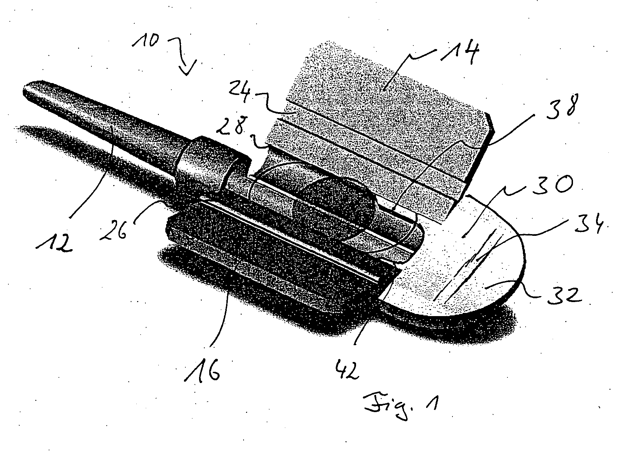

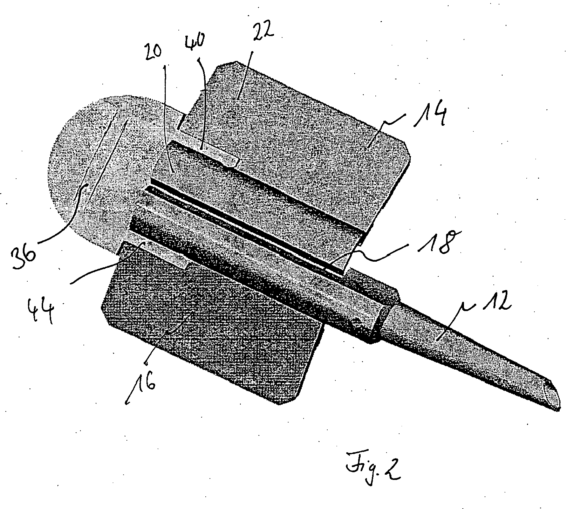

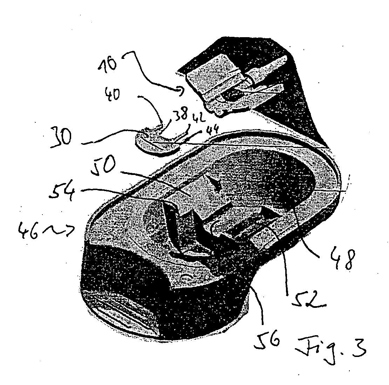

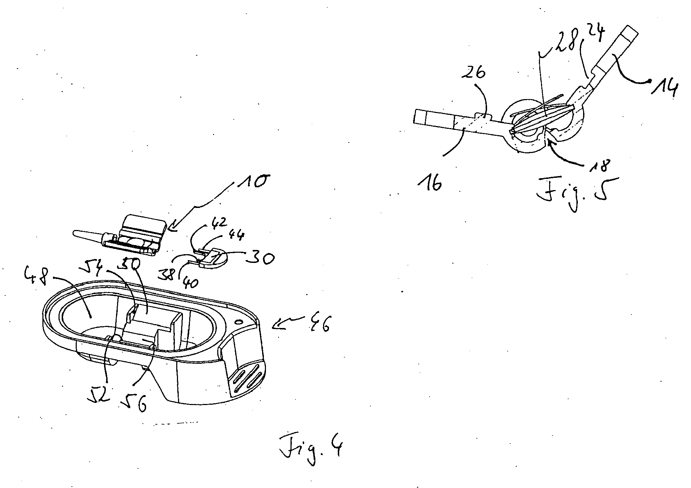

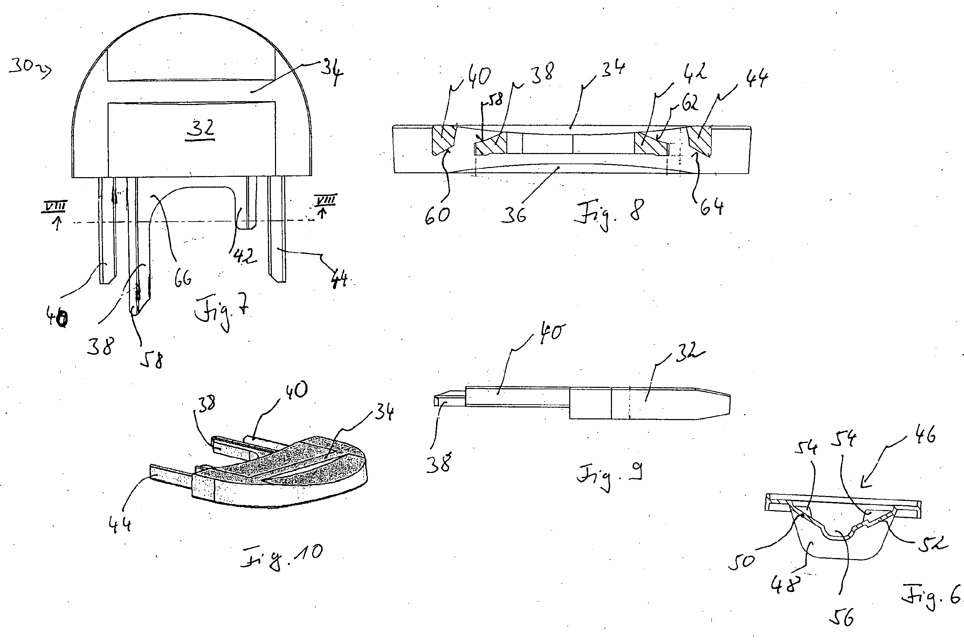

Fign. 1 und 5 zeigen eine erfindungsgemäße Kartusche 10, die gelegentlich auch als Cartridge bezeichnet wird. Die Kartusche besitzt eine hohle Injektionsspitze 12 und zwei Flügelelemente 14 und 16. Die Ränder an der Austrittsöffnung der Injektionsspitze sind abgerundet. Wie auch in Fig. 2 zu erkennen, ist das Flügelelement 14 über ein Filmscharnier 18 an dem Flügelelement 16 angelenkt.

Jedes Flügelelement besitzt einen bogenförmigen Abschnitt 20, an den sich ein ebener Abschnitt 22 anschließt. Auf den einander zugewandten Seiten sind die ebenen Abschnitte der Flügelelemente mit einem Vorsprung 26 und einer Ausnehmung 24 ausgebildet. Die bogenförmigen Abschnitte der Flügelelemente bilden in der geschlossenen Position eine Verlängerung eines Linsenkanals, über den die Linse appliziert wird.

In Fig. 1 ist eine zwischen den Flügelelementen 14 und 16 gehaltene Intraokularlinse 28 dargestellt. Das Beispiel aus Fig. 3 zeigt eine dreiteilige Intraokularlinse. Die Kartusche läßt sich aber ebenfalls für einteilige Intraokularlinsen verwenden.

Im Übergangsbereich zwischen gewölbtem Abschnitt 20 und ebenem Abschnitt 22 besitzt die Kartusche einen Hinterschnitt 28, an dem die Haptik der Linse und ggf. auch der Linsenkörper anliegt, um die Linse in der Kartusche zu halten.

Die in den Figuren dargestellte Position der Kartusche wird durch ein Sperrelement 30 gehalten. Das Sperrelement 30 ist in den Figuren 1 und 2 in der aufgesteckten Position dargestellt und in den Figuren 7 bis 9 als isoliertes Teil. Das Sperrelement 30 besitzt einen Griff 32, der flach ausgebildet ist und auf der Vorder- und Rückseite einen quer zur Längsrichtung der Kartusche verlaufenden Steg 34, 36 besitzt.

An den Griff 32 schließen sich zwei Fingerpaare an. Ein erstes Fingerpaar besitzt einen Finger 38 und einen zugehörigen zweiten Finger 40. Finger 42 bildet den innen liegenden Finger des anderen Fingerpaares, das das Flügelelement 16 in der geöffneten Position sichert. Wie in Fig. 1 dargestellt, sind die jeweils innen liegenden Finger 38, 42 der Fingerpaare im gewölbten Abschnitt des Flügelelements unterhalb von dem Hinterschnitt 28 angeordnet, während die außen liegenden Finger 40, 44 im wesentlichen jeweils auf dem ebenen Abschnitt des Flügelelements angrenzend an den gewölbten Abschnitt angeordnet sind. Das aufgesteckte Sperrelement 30 hält die Kartusche in der' dargestellten Position, in der die Flügelelemente aufgespreizt sind.

Figur 7 zeigt eine Draufsicht auf das Sperrelement 30. Deutlich zu erkennen ist, daß die Fingerpaare 38, 40 und 42, 44 jeweils eine unterschiedliche Länge besitzen. Das erste Fingerpaar 38, 40 ist derart ausgebildet, daß der äußere Finger 40 kürzer ist als der innere Finger 38. Bei dem zweiten Fingerpaar sind die Längenverhältnisse genau umgekehrt, sodaß der innere Finger kürzer ist als der äußere Finger. Beide äußeren Finger 40 und 44 besitzen ungefähr die gleiche Länge. Die Länge des Fingers 38 ist so bemessen, dass das freie Ende des Fingers 38 eine Anschlagfläche für die Linse bildet und ein Zurückschieben dieser verhindert.

Das erste Fingerpaar ist somit insgesamt länger, wobei das erste Fingerpaar das angelenkte Flügelelement 14 hält.

Die inneren Finger 38 und 42 besitzen ― trotz ihrer unterschiedlichen Länge ― einen gleichen Querschnitt, wie aus Figur 8 ersichtlich. Die Finger 38 und 40 besitzen zwei im wesentlichen parallel zueinander angeordnete, einander zugewandte Anlageflächen 58 und 60. Figur 8 zeigt den entspannten Zustand des Sperrelements. Im aufgesetzten Zustand sind die inneren Finger 38 und 42 relativ zu den äußeren Fingern 40 und 44 versetzt, sodaß die Anlageflächen 58 und 60 an der Kartuschenwand anliegen. Das zweite Fingerpaar 42 und 44 besitzt entgegengesetzt geneigte Anlageflächen 62 und 64, die am dementsprechend anderen Flügelelement anliegen.

Für die inneren Finger 38 und 42 ist in dem Zwischenbereich zwischen den Fingern zusätzlich ein Stabilisierungsabschnitt 66 vorgesehen.

Fign. 3 und 4 zeigen in einer explosionsartig auseinandergezogenen Darstellung die Kartusche 10 mit dem Sperrelement 30. Aufbewahrt und transportiert wird die Kartusche 10 in einem Behälter 46. Der Behälter besitzt eine wannenförmige Vertiefung 48, in der eine Halteeinrichtung für die Kartusche 10 vorgesehen ist. Die Halteeinrichtung besitzt zwei in einem schrägen Winkel zueinander angeordnete Ablageflächen 50, 52, die seitlich durch Haltevorsprünge 54 begrenzt sind. Zwischen den Flächen 50, 52 ist eine Vertiefung 56 vorgesehen, die die gewölbten Abschnitte der Flügelelemente aufnimmt.

Die in den Behälter eingesetzte Kartusche wird durch eine Folie abgedeckt, die vor der Benutzung von dem Behälter abgezogen wird. Der Behälter ist aus PET hergestellt und durch Tiefziehen geformt. Für eine Lagerung oder einen Transport der Linse ist der Behälter 46 mit einer Flüssigkeit gefüllt, die eine Veränderung der Linse verhindert. Im Gebrauch kann die Kartusche 10 dann mit einer Pinzette an dem Griffelement aus dem Behälter herausgenommen werden. Die Linse wird in der Kartusche nachfolgend benetzt. Zum Schließen der Kartusche werden die beiden Flügelenden mit den Fingerspitzen zusammengebracht, nachdem das Sperrelement 30 aus der Kartusche herausgezogen wurde. Der Hinterschnitt 28 stellt herbei sicher, daß die Linse in der Kartusche sich in der richtigen Ausrichtung zusammenfaltet. Die so verschlossene Kartusche wird anschließend in einen Injektor eingesetzt, von wo aus die Linse über einen Stößel des Injektionssystems durch den Linsenkanal herausgeschoben wird.

## Patentansprüche

1. Vorrichtung zum Laden von Intraokularlinsen in ein Injektionssystem, mit
- einer Kartusche (10), die zwei Flügelelemente (14, 16) aufweist, von denen mindestens eines der beiden schwenkbar angelenkt ist, die Flügelelemente bilden in einer geschlossenen Position einen Linsenkanal für die gefaltete Intraokularlinse,
**dadurch gekennzeichnet, daß**
ein Sperrelement (30) vorgesehen ist, das mit den Flügelelementen (14, 16) zusammenwirkt, um die Kartusche (10) in einer Position mit geöffneten Flügelelementen zu halten und
das Sperrelement einen Griffabschnitt (32) und einen Halteabschnitt (38, 40, 42, 44) besitzt.

2. Vorrichtung nach Anspruch 1, **dadurch gekennzeichnet, daß** das Sperrelement (30) scheibenförmig ausgebildet ist.

3. Vorrichtung nach Anspruch 2, **dadurch gekennzeichnet, daß** der Halteabschnitt an einer Seite des Griffelements mindestens zwei Paare von Fingern (38, 40, 42, 44) aufweist, von denen jeweils ein Paar ein Flügelelement hält.

4. Vorrichtung nach Anspruch 3, **dadurch gekennzeichnet, daß** die Finger jeweils einen geraden, länglichen Schlitz bilden, mit dem das Sperrelement auf die Kartusche auf- und/oder von dieser abgezogen wird.

5. Vorrichtung nach Anspruch 3 oder 4, **dadurch gekennzeichnet, daß** das Halteelement an dem Griffabschnitt eine gerade Seite besitzt, von der die Fingerpaare mit parallel ausgerichteten Schlitzen abstehen.

6. Vorrichtung nach einem der Ansprüche 1 bis 5, **dadurch gekennzeichnet, daß** zusätzlich ein wannenförmiger Behälter für die Kartusche vorgesehen ist.

7. Vorrichtung nach Anspruch 6, **dadurch gekennzeichnet, daß** der Behälter zwei Ablageflächen (50, 52) für die Flügelelemente in der geöffneten Position der Kartusche besitzt.

8. Vorrichtung nach Anspruch 7, **dadurch gekennzeichnet, daß** die Ablageflächen seitlich Halteabschnitte für die Flügelelemente besitzen.

9. Vorrichtung nach Anspruch 7 oder 8, **dadurch gekennzeichnet, daß** die Ablageflächen in einer Vertiefung angeordnet sind derart, daß für eine auf den Ablageflächen abgelegte Kartusche sowohl eine Kartuschenspitze als auch das Halteelement seitlich vorstehen.

10. Vorrichtung nach einem der Ansprüche 6 bis 9, **dadurch gekennzeichnet, daß** der Behälter einstückig geformt ist.

11. Vorrichtung nach Anspruch 10, **dadurch gekennzeichnet, daß** der Behälter durch eine Abdeckung flüssigkeitsdicht verschlossen ist.

12. Vorrichtung nach Anspruch 11, **dadurch gekennzeichnet, daß** als Abdeckung eine abziehbare Folie vorgesehen ist.

13. Vorrichtung nach einem der Ansprüche 1 bis 12, **dadurch gekennzeichnet, daß** die Flügelelemente auf ihren einander zugewandten Seiten einen Verschluß aufweisen.

14. Vorrichtung nach Anspruch 13, **dadurch gekennzeichnet, daß** der Verschluß mindestens einen Vorsprung und mindestens eine Vertiefung aufweist, die in einer geschlossenen Position der Kartusche ineinander angeordnet sind.

15. Vorrichtung nach Anspruch 14, **dadurch gekennzeichnet, daß** der Vorsprung sich entlang dem Flügelelement parallel zur Schwenkachse des Flügelelements erstreckt.

16. Vorrichtung nach einem der Ansprüche 1 bis 15, **dadurch gekennzeichnet, daß** jedes Flügelelement einen kreisförmigen gewölbten Abschnitt aufweist, der in einen ebenen Abschnitt übergeht.

17. Vorrichtung nach einem der Ansprüche 1 bis 16, **dadurch gekennzeichnet, daß** die Kartusche eine abgerundete Kartuschenspitze aufweist.
